Europäisches Patentamt

European Patent Office

Office européen des brevets

(19)

(11) Publication number: **0 073 029**
**B1**

(12) # EUROPEAN PATENT SPECIFICATION

(45) Date of publication of patent specification: **11.11.87**

(21) Application number: **82107601.5**

(22) Date of filing: **19.08.82**

(51) Int. Cl.⁴: **C 12 N 15/00,** C 12 N 9/50,
C 12 N 1/20 // C12R1/19

(54) Recombinant plasmid and microorganism containing same.

(30) Priority: **24.08.81 JP 131631/81**

(43) Date of publication of application:
**02.03.83 Bulletin 83/09**

(45) Publication of the grant of the patent:
**11.11.87 Bulletin 87/46**

(84) Designated Contracting States:
**AT BE CH DE FR GB IT LI LU NL SE**

(56) References cited:
**EP-A-0 057 350**

**AGRIC. BIOL. CHEM., vol. 44, no. 6, 1980;
H.UCHIYAMA et al.: "Purification of prorennin
mRNA and it's translation in vitro", pp. 1373-
1381**

**JOURNAL OF BIOCHEMISTRY, vol. 90, no. 3,
1981; K.NISHIMORI et al.: "Cloning in
Escherichia coli of the structural gene of
prorennin, the precursor of calf milk-clotting
enzyme rennin", pp. 901-904**

**JOURNAL OF BIOCHEMISTRY, vol. 91, no. 3,
1982; K.NISHIMORI et al.: "Nucleotide
sequence of calf prorennin cDNA cloned in
Escherichia coli", pp. 1085-1088**

(73) Proprietor: **Beppu, Teruhiko
No. 5-21, 1-chome
Horinouchi Suginami-ku, Tokyo (JP)**

(72) Inventor: **Beppu, Teruhiko
1-5-21, Horinouchi
Suginami-ku Tokyo (JP)**
Inventor: **Uozumi, Takeshi
4-32-8, Takashimadaira
Itabashi-ku (JP)**
Inventor: **Nishimori, Katsuhiko
603, Mukogaoka Heights 1-5-4, Mukogaoka
Bunkyo-ku Tokyo (JP)**

(74) Representative: **Henkel, Feiler, Hänzel & Partner
Möhlstrasse 37
D-8000 München 80 (DE)**

(56) References cited:

**NUCLEIC ACIDS RESEARCH, vol. 10, no. 7,
1982, IRL Press Ltd., London (GB);
T.J.R.HARRIS et al.: "Molecular cloning and
nucleotide sequence of cDNA coding for calf
preprochymosin", pp. 2177-2187**

## Description

This invention relates to a recombinant plasmid and a host microorganism containing same, which microorganism is capable of producing calf prorennin not containing the first 5 amino acids. More specifically, this invention relates to a recombinant plasmid comprising cDNA (complementary DNA) of calf prorennin.

Calf prorennin is a proenzyme protein secreted by the fourth stomach of the calf within about a few weeks after birth, and it is the precursor of the milk-coagulating enzyme, rennin (chymosin), indispensable in a process for making cheese. Rennin is excreted from the mucosa of the fourth stomach of calf and is an acidic protease having 323 amino acid residues. Prorennin, the precursor of rennin, is an enzyme protein having 365 amino acid residues and a molecular weight of about 40,000 and it is converted to the milk-coagulating enzyme rennin autocatalytically under acidic conditions.

In recent years, the production of rennin has not been able to meet demands, and a substitute microbial rennet is being used. Accordingly, there is great industrial significance in the supply of a large amount of rennin or prorennin.

Recombinant DNA technology refers to the technique of introducing into the vector-plasmid of a suitable host microorganism, cDNA having genetic information by which a useful substance is produced; forming a recombinant plasmid; and utilizing said host organism containing the recombinant plasmid to produce said useful substance. Such recombinant DNA technology has progressed rapidly in recent years, and for instance, a technique of producing useful substances such as growth hormone, interferon, etc. by *Escherichia coli* as the host microorganism is under development.

Beppu, one of the present inventors, and others have been investigating the cloning of the structural gene of prorennin in all sequences. The works have been disclosed in the following papers; "Abstracts" of the Annual Meeting of the Japanese Agricultural Chemistry Society," p408 and 1980, Proceedings of the IVth International Fermentation Symposium, p179 F-21-5(L) in 1980, "Abstracts" of the Annual Meeting of the Japanese Agricultural Chemistry Society, p259 and p569 in 1981, and Agric. Biol. Chem. *44* (6), p. 1373—1381 (1980).

However, the present inventors have not successfully completed the cloning of clones which have essentially all the sequence of the structural gene of prorennin and are expected to be capable of producing calf prorennin, the presence of which clones can be confirmed by the hybrid-arrested translation assay method. In the clonings in the above-reported literatures, strains displaying positive hybridization have been obtained; nevertheless, they do not contain the entire cDNA of prorennin but the partial segment of the cDNA having the partial sequence of the structural gene of prorennin with a length of about 0.2Md.

As a result of further studies that will be explained in detail in Examples of this specification, the inventors have now succeeded in preparing clones which contain a recombinant plasmid having essentially all the sequence of the prorennin structural gene as confirmed in the hybrid-arrested translation assay and are capable of producing calf prorennin according to the invention.

The inventors, using the method that will be detailed later, have succeeded in constructing a recombinant plasmid comprising calf prorennin cDNA and in obtaining host microorganisms having this recombinant plasmid. Novel microorganisms having this novel recombinant plasmid are those belonging to the P2 level (deposition of which is not accepted by Fermentation Research Institute, Agency of Industrial Science and Technology Japan) and one species, for instance, *Escherichia coli* C 600 $r_k^- m_d^-$ (pTACR1), which is stored at Fermentation Laboratory, Agricultural Chemistry Department, Faculty of Agriculture, the University of Tokyo.

The purpose of this invention is to provide a recombinant plasmid comprising calf prorennin cDNA and furthermore, a host microorganism having said recombinant plasmid.

Another purpose of this invention is to provide a process for producing calf prorennin not containing the first 5 amino acids which comprises cultivating in a nutrient medium, a genetically engineered microorganism and isolating calf prorennin from the culture broth, said genetically engineered microorganism containing a recombinant plasmid composed of calf prorennin cDNA.

The above purposes of this invention as well as advantages thereof will become clearer in the following description.

According to this invention, prorennin mRNA (messenger RNA) is extracted from the fourth stomach of the calf within a few weeks after birth and a complementary single-stranded DNA is synthesized using the reverse transcriptase method with said mRNA as template, which template is then removed by alkaline treatment. From the single-stranded DNA obtained above, a double-stranded cDNA is synthesized using, for instance, DNA polymerase I or reverse transcriptase. By the method utilizing nuclease S1, only the hair-pin structure is removed selectively from the double-stranded cDNA to form the double-stranded cDNA. Then, to both 3' ends thereof is attached poly dG(deoxyguanine) homopolymer according to the TdT(terminal deoxynucleotidyl transferase) method.

On the other hand, a suitable vector plasmid such as *E. coli* vector pBR322 is treated with restriction enzyme, *Sal* I to furnish linear pBR322 DNA and then both 3' ends of a single-stranded portion of the linear pBR322 are repaired by using the DNA polymerase I method. To the repaired 3' ends is attached poly(d-C)-homopolymer according to the TdT method.

The linear pBR322 DNA with the attachment of poly(dC)hompolymer at the 3' ends is mixed with the double-stranded cDNA having the attachment of poly(dG)-homopolymer at the 3' ends, and the mixture is annealed to form a recombinant plasmid comprising calf prorennin cDNA.

Then, *E. coli* is treated with calcium salts, preferably at a cold temperature and mixed with the recombinant plasmid to form transformant strains of *E. coli*.

Clones having some inserted DNA at the *Sal* I site are selected, according to the method utilizing drug-resistance of vector plasmids. In order to choose clones having prorennin cDNA among the selected ones above, colony hybridization is performed using as probe a radioactively-labelled prorennin-specific mRNA such as [125]I-mRNA or $^{32}$P-mRNA. Candidate strains of host microorganism possibly comprising calf prorennin cDNA are screened by the hydrid-arrested translation method for the presence of the inserted structureal gene of prorennin. As will be shown in the Examples later, the present invention has provided clones having substantially all the sequence of the prorennin structural gene as confirmed by the hybrid-arrested translation method. Furthermore, according to the method of Maxam and Gilbert, the presence of base sequence corresponding to the amino acid sequence of prorennin N terminus has been confirmed.

Although in the preceding description the inventors have disclosed one embodiment of obtaining recombinant plasmid and host microorganism containing same, several variations can be adopted with respect to the embodiment, such as in choosing host microorganism or vector plasmid and in synthesizing recombinant plasmid. As other examples of the host microorganism, yeast such as *Saccharomyces cerevisiae*, gram-positive bacteria (e.g. *Bacillus subtilis*), and gram-negative bacteria (e.g. *Pseudomonas*) can be exemplified. For instance, the method for synthesizing a recombinant plasmid using a restriction enzyme, endonuclease-ligase, is also applicable in this invention. Other examples of the vector plasmid are as follows: when *E. coli* is the host microorganism, known vector plasmids such as pMB9 are used: when the host is yeast, various known vector plasmids derived from 2µDNA or yeast chromosone are used; when the host is *B. subtilis*, known vector plasmids such as pUB101 are used; and when the host is *Pseudomonas* bacterium, various plasmids having drug-resistance are used.

An embodiment of this invention will be illustrated further in the following.

I. Isolation of Prorennin mRNA

Prorennin mRNA is extracted from the mucosa of the fourth stomach of a calf within about a few weeks after birth. Extraction can be carried out according to a modification of Uchiyama's method (H. Uchiyama, T. Uozumi, T. Beppu, and K. Arima, Agr. Biol. Chem. *44*, p. 1373—1381, 1980). For instance, the mucosa of the fourth stomach of calf are collected and powdered at low temperature, from which all RNA is extracted by the phenol method, preferably in the presence of bentonite. An aqueous extract containing polymer RNA is treated with a precipitating agent such as lithium chloride and the precipitate containing the polymer RNA is collected. From an aqueous solution containing the polymer RNA precipitates thus prepared, poly(A) tailed RNA fractions containing prorennin mRNA can be separated and collected by affinity-chromotography using an absorbent such as poly(U)-Sapharose or oligo(dT)Sepharose. Namely, the poly(A)mRNA fractions containing prorennin mRNA are selectively adsorbed on the adsorbent, and ribosomal RNA and other impurities are eluted and removed through the adsorbent column. Elution with an aqueous formamide solution gives an aqueous formamide solution containing prorennin mRNA. Preferably, the above-stated operations are repeated several times.

In accordance with a known procedure, the fractions containing prorennin mRNA are subjected to the sucrose density gradient centrifugation and by such molecular weight fractionation, fractions (prorennin mRNA fractions) having a molecular weight centered at a sedimentation constant of 15s can be collected. The mRNA fractions are tested by *in vitro* protein synthesis for their coding activity. These fractionating operations can be repeated a number of times if desired.

II. Preparation of Prorennin cDNA

Using the reverse transcriptase method with the above prorennin mRNA as template, the complementary single-stranded DNA of said mRNA is synthesized and the single-stranded cDNA of prorennin can then be obtained after removal of the template mRNA by alkaline treatment. For instance, said prorennin mRNA is reacted with the reverse transcriptase in the presence of four kinds of deoxynucleotide triphosphates (dXTP's) and also oligo(dT) as a primer, and the complementary single-stranded DNA complexed with said prorennin mRNA can be synthesized. The resulting material is heated at around 70°C in the presence of an alkali to remove the template mRNA by destruction and thus the single-stranded prorennin cDNA is produced.

The single-stranded prorennin cDNA is then reacted with DNA polymerase I or a reverse transcriptase in the presence of dXTP's to form a complementary double-stranded cDNA. Since the double-stranded cDNA has a hair-pin structure, the cDNA is reacted with nuclease S1 that is capable of selectively cutting off the hairpin structure and after removal thereof, the complete prorennin-specific double-stranded cDNA can be synthesized.

III. Ligation of Prorennin cDNA and vector plasmid

Complementary deoxynucleotidyl homopolymer is attached to both 3' ends of the above synthesized complete prorennin-specific double-stranded cDNA. A suitable host vector plasmid is cut at desired sites and to the resulting linear DNA is attached the homopolymer at both 3′ ends.

For instance, poly(dG)homopolymer can be attached to both 3′ ends of said prorennin double-stranded cDNA by the reaction with TdT in the presence of dGTP (deoxyguanosine triphosphate). On the other hand, a suitable vector plasmid such as vector plasmid pBR322 of *E. coli* is reacted with restriction enzyme *Sal* I endonuclease to form linear pBR322 DNA. Both 3′ ends of the single-stranded portion of the linear plasmid DNA are repaired by the reaction with DNA polymerase I in the presence of four kinds of deoxynucleotide triphosphates (dXTP's) and to the repaired ends can be attached poly (dC) homopolymer by the reaction with TdT in the presence of deoxycytosine triphosphate (dCTP).

According to this invention, a recombinant plasmid containing calf prorennin cDNA can be synthesized in the following manner. The prorennin-specific double-stranded cDNA having poly(dG)homopolymer attached at 3′ ends such as the one prepared above, is mixed with the linear pBR322 DNA having poly(dC)homopolymer attached at the repaired 3′ ends, and upon annealing the mixture both DNA are linked at the poly(dC) tail of the latter DNA and the complementary poly(dG) tail of the former. Thus the recombinant plasmid containing essentially the entire length of the prorennin structural gene can be synthesized.

IV. Transformation of host microorganism and detection of clones containing recombinant plasmid comprising calf prorennin cDNA

A host microorganism such as *E. coli* C 600 $r^-m^-$ is treated with calcium salts and then mixed with the recombinant plasmid comprising calf prorennin cDNA to incorporate said recombinant plasmid into *E. coli*, thus transformants being prepared. Clones with the recombinant plasmid comprising prorennin cDNA are selected from the transformants.

For instance, when prorennin cDNA is inserted at the *Sal* I site of pBR322, the transformants with the recombinant plasmid comprising calf prorennin cDNA are expected to be ampicillin-resistant and tetracycline-sensitive. In a preliminary manner, the desired transformants can be distinguished from those that are ampicillin-resistant and tetracycline-resistant due to the difference in resistance against ampicillin and tetracycline. In order to separate the real transformants having the recombinant plasmid from those that are expected to have same, colony hybridization and hybrid-arrested translation are carried out. The colony hybridization can be performed according to a known method where the prorennin-specific mRNA is labelled with radioactive $^{125}$I or $^{32}$P and $^{125}$I-mRNA or $^{32}$P-mRNA is used as probe. By this colony hybridization, clones displaying positive hybridization are identified and they are further tested by the known hybrid-arrested translation for the presence of the inserted structural gene of prorennin.

The following examples will exemplify the practice of the invention. They are presented for illustrative purposes only, and should not be construed as limiting the invention in any way.

Example
(1) Extraction and purification of prorennin messenger RNA (mRNA)

Mucous membrane of two calf abomasums (two weeks after birth) (ca. 140 g) was peeled off; washed with a cooled Tris-Hcl buffer solution (0.1M, pH 9.0) containing 0.025 M EDTA, 0.1M NaCl, and 1% SDS, dehydrated in acetone-dry ice; frozen; and powdered with a homogenizer. The powders were suspended in the buffer solution (1l) containing bentonite and to this was added a mixed solvent (1l) comprising phenol, chloroform, and isoamyl alcohol (50:50:1) and after stirring, it was centrifuged and the upper aqueous phase was separated. After repeating same operations six times, lithium chloride was added to the final aqueous phase to make the final concentration 2M. Upon standing overnight at 4°C, precipitates were collected by centrifugation and they were dissolved in 60 ml of 0.1×SSC (150 mM of NaCl and 15 mM sodium citrate). The same operations were repeated once again and to the resulting solution were added two volumes of ethanol to form precipitation of polymer RNA, which was repeated three times.

The final precipitate was dissolved in water (6 ml) and to this was added 4 volumes of a Tris-Hcl buffer solution (pH 7.5) containing 0.01M EDTA, 0.7M NaCl, and 25% formamide. The solution was passed through poly (U) Sepharose 4B column (volume, 30 ml) and the column was washed with the above buffer solution (30 ml). After washing with 30 ml of the buffer solution, the adsorbed mRNA was eluted with an aqueous solution (pH 7.5) containing 0.01M EDTA, 0.2% SDS, 90% formamide, and 0.01M potassium phosphate. The eluate was dialyzed against distilled water and on addition of ethanol, RNA containing mRNA was precipitated. The precipitate was, after dissolving, centrifuged for 13 hours at 50,000×g in 5 to 20% sucrose density gradients. Fractions containing prorennin mRNA (120 μg of RNA with a molecular weight centered at 15s) were collected. These fractions were tested *in vitro* in protein synthesis system using rabbit reticulocyte lysate and it was confirmed that the fractions have such coding activity that can produce prorennin and the amount thereof exceeds 70% of the total translational products.

(2) Preparation of prorennin cDNA

In order to synthesize single-stranded cDNA by using the prorennin mRNA as template, a solution (400 μl) containing the following ingredients was incubated at 42°C for 1h; these are mRNA (30 μ), AMV reverse transcriptase (140 units), oligo(dT) 12—13 (1.6 μ), four kinds of deoxynucleotide triphosphates (each 0.5 mM), Actinomycin D(40 μg), NaCl (60 mM), $MgCl_2$ (6 mM), DTT (2 mM), and Tris-Hcl (50 mM, pH 8.3). The reaction mixture was extracted with phenol, precipitated with ethanol, dissolved in 0.3N NaOH, and heated at 68°C for 15 min to destroy the template mRNA. Subsequent gel filtration through Sephadex G-50 gave 240 ng of the single-stranded cDNA. In order to synthesize double-stranded cDNA by using the above single-stranded cDNA, a solution (240 µl) containing the following ingredients was incubated at 25°C for 4h; these are the single-stranded cDNA (200 ng), DNA polymerase fragment A (18 units), four kinds of deoxynucleotide triphosphates (each 1 mM), $MgCl_2$ (4 mM), β-mercaptoethanol (0.5 mM), and Tris-HCl (30 mM, pH 7.5). The reaction product was extracted with phenol and precipitated with ethanol. The precipitates were treated with 80 units of nuclease Sl at 40°C for 90 min in a solution (200 µl, pH 4.6) containing sodium acetate (30 mM), NaCl (50 mM), and $ZnSO_4$ (1 mM) and the hair-pin structure were removed by decomposition. The resultant product was centrifuged in from 5 to 30% sucrose density gradients at 95,000 xg for 12h to produce 100 ng of the double-stranded cDNA having a mean molecular weight of 0.7 Md.

(3) Annealing of prorennin cDNA and vector plasmid

The above prorennin cDNA (0.05 p mole) was incubated at 37°C for 6 min in a solution A (120 µl) containing 80 units of terminal deoxynucleotidyl transferase, dGTP (0.06 µmole), $MgCl_2$ (5 mM), and HEPES-NaOH (10 mM, pH 7.1); and (dG) homopolymer was thus attached to 3' ends of the cDNA.

On the other hand, vector plasmid pBR322 was cut by Sal I, endonuclease to form linear DNA and the linear DNA was incubated with DNA polymerase (fragment A) in the presence of four kinds of deoxynucleotide triphosphates to repair the single-stranded ends. The DNA thus obtained (0.5 p mole) was incubated at 25°C for 10 min in a solution B (120 µl) containing 24 units of terminal deoxynucleotidyl transferase, dCTP (0.04 µ mole), $CoCl_2$ (1 mM), potassium cacodylate (100 mM), and Tris-HCl (30 mM, pH 7.6); and (dC) homopolymer was thus attached to 3' end of the DNA.

Reaction mixture A (120 µl) and reaction mixture B (24 µl) were mixed together at 0°C after stopping incubations in both mixtures A and B by the addition of EDTA. The product was extracted with phenol and precipitated with ethanol. The precipitate was dissolved in a 100 mM Tris-HCl solution (pH 7.6) containing EDTA (1 mM) and NaCl (100 mM) at a concentration of 0.3 µg per ml and it was heated at 68°C for 2h and gradually cooled to room temperature at a cooling rate of 5°C per 30 min. Thus the prorennin cDNA having (dG) homopolymer attached and pBR322 DNA having (dC) homopolymer attached were linked by annealing.

(4) Transformation of *E. coli* with annealed DNA and selection of clones containing prorennin cDNA

Transformation of *E. coli* by the use of the annealed DNA obtainable in process (3) was performed according to a conventional method involving the use of calcium treatment. *E. coli* C600 r⁻m⁻ transformed accordingly and 804 transformants were selected on L broth agar plates containing 30 µg/ml ampicillin. Then, among 804 colonies, transformants that were ampicillin-resistant and tetracycline-sensitive on the same medium containing 10 µg/ml of tetracycline were selected. The total number of such colonies was 622. The 622 transformants were implanted on Millipore filters in a lattice fashion that were attached onto the L broth agar medium containing 30 µg/ml of ampicillin. Colonies grown on the filters were washed, treated with protease, and heated *in vacuo* at 80°C for 2h after treatment with 95% ethanol and chloroform, thus fixing DNA of each colony onto the filters.

Three filters were prepared simultaneously and each filter was subjected to colony hybridization in the following manner; filter (1) was hybridized with the prorennin mRNA labelled with [125]I as probe; filter (2) was, after treatment with excessive ribosomal rRNA, hybridized with the same [125]I-mRNA; and filter (3) was hybridized with [125]I-rRNA. By comparing all three filters, clones containing rDNA were first removed and thirty clones presumably containing prorennin cDNA were obtained. Recombinant plasmids (DNA) were obtained from these thirty clones by CsCl-ethidium bromide equilibrium centrifugation. Each plasmid was hybridized with the prorennin mRNA obtained from the previous process (1) and tested for *in vitro* protein synthesis in a reticulocyte lysate system to see if it inhibits the prorennin synthesis. By this hybrid-arrested translation method, ten clones were determined to contain the prorennin cDNA.

(5) Characteristics of the cloned prorennin cDNA

pTACR1 plasmid DNA was extracted from one of the clones (102-B strain), treated with Sal I endonuclease, and subjected to agarose gel electrophoresis. Thus inserted DNA fragment corresponding to approximately the entire length of prorennin cDNA was detected to be present. The base sequence of this inserted DNA fragment was determined according to the method of Maxam and Gilbert. As will be evident in the following, the base sequence corresponding to the amino acid sequence prorennin N terminus has been proved to be present in the inserted DNA fragment.

5'-GCT GAG ATC ACT AGG ATC CCT CTG TAC AAA GGC-3'

Ala Glu Ile Thr Arg Ile Pro Leu Tyr Lys Gly

The plasmid DNAs obtained from the clones definitely containing the prorennin cDNA was analyzed according to the Maxam-Gilbert method for its base sequence. As a result of the base sequence determination, it was found that out of 1095 bases corresponding to the entire sequence of the prorennin cDNA, plasmid pTACR 102B6 comprises from the 1st base corresponding to the prorennin N terminus up to the 890th base and plasmid pTACR 103C2 comprises from the 771st base up to the 1095th base corresponding to the prorennin C terminus.

Plasmid pCR 1001 containing the entire length of prorennin cDNA was constructed in the following manner. pTACR 102B6 and pTACR 103C2 were treated with *Sal* I to obtain DNA fragments. Each DNA fragment was digested with exonuclease III to cut off about 150 bases from both 3' ends. The fragment derived from pTACR 102B6 was cut with *Kpn* I and the fragment derived from pTACR 103C2 was cut with *Bam* HI, respectively. Both fragments were annealed thereafter to produce fragment (a). Independently, small fragment (b) was prepared by the treatment of pTACR 102B6 with *Eco* RI plus *Kpn* I. In a similar manner, large fragment (c) was prepared by the treatment of pTACR 103C2 with *Eco* RI plus *Bam* HI. All three fragments, (a), (b) and (c) were mixed, annealed and linked by T4 DNA ligase. pCR1001 thus constructed contains the complete cDNA corresponding to all the amino acid sequence of prorennin. The base sequence including the stopping codon thereof is illustrated below.

```
1                        20                      40
|                        |                       |
GCT GAG ATC ACC AGG ATC CCT CTG TAC AAA GGC AAG TCT CTG AGG AAG GCG CTG AAG
Ala Glu Ile Thr Arg Ile Pro Leu Tyr Lys Gly Lys Ser Leu Arg Lys Ala Leu Lys
                                  10

    60                           80                      100
    |                            |                       |
GAG CAT GGG CTT CTG GAG GAC TTC CTG CAG AAA CAG CAG TAT GGC ATC AGC AGC AAG
Glu His Gly Leu Leu Glu Asp Phe Leu Gln Lys Gln Gln Tyr Gly Ile Ser Ser Lys
20                                      30

      120                          140                        160
      |                            |                          |
TAC TCC GGC TTC GGG GAG GTG GCC AGC GTG CCC CTG ACC AAC TAC CTG GAT AGT CAG
Tyr Ser Gly Phe Gly Glu Val Ala Ser Val Pro Leu Thr Asn Tyr Leu Asp Ser Gln
    40                                      50

      180                          200                        220
      |                            |                          |
TAC TTT GGG AAG ATC TAC CTC GGG ACC CCG CCC CAG GAG TTC ACC GTG CTG TTT GAC
Tyr Phe Gly Lys Ilu Tyr Leu Gly Thr Pro Pro Gln Glu Phe Thr Val Leu Phe Asp
    60                                      70

          240                          260                        280
          |                            |                          |
ACT GGC TCC TCT GAC TTC TGG GTA CCC TCT ATC TAC TGC AAG AGC AAT GCC TGC AAA
Thr Gly Ser Ser Asp Phe Trp Val Pro Ser Ile Tyr Cys Lys Ser Asn Ala Cys Lys
            80                                      90

          300                          320                        340
          |                            |                          |
AAC CAC CAG CGC TTC GAC CCG AGA AAG TCG TCC ACC TTC CAG AAC CTG GGC AAG CCC
Asn His Gln Arg Phe Asp Pro Arg Lys Ser Ser Thr Phe Gln Asn Leu Gly Lys Pro
          100                                     100

          360                          380
          |                            |
CTG TCT ATC CAC TAC GGG ACA GGC AGC ATG CAG GGC ATC CTG GGC TAT GAC ACC GTC
Leu Ser Ile His Tyr Gly Thr Gly Ser Met Gln Gly Ile Leu Gly Thr Asp Thr Val
              120                                     130

400                          420                        440
|                            |                          |
ACT GTC TCC AAC ATT GTG GAC ATC CAG CAG ACA GTA GGC CTG AGC ACC CAG GAG CCC
Thr Val Ser Asn Ile Val Asp Ile Gln Gln Thr Val Gly Leu Ser Thr Gln Glu Pro
              140                                     150
```

```
        460                          480                              500
         |                            |                                |
GGG GAC GTC TTC ACC TAT GCC GAA TTC GAC GGG ATC CTG GGG ATG GCC TAC CCC TCG
Gly Asp Val Phe Thr Tyr Ala Glu Phe Asp Gly Ile Leu Gly Met Ala Tyr Pro Ser
                            160                                      170

            520                          540                          560
             :                            !                            !
CTC GCC TCA GAG TAC TCG ATA CCC GTG TTT GAC AAC ATG ATG AAC AGG CAC CTG GTG
Leu Ala Ser Glu Tyr Ser Ile Pro Val Phe Asp Asn Met Met Asn Arg His Leu Val
                                180                                      190

                580                          600                          620
                 |                            |                            |
GCC CAA GAC CTG TTC TCG GTT TAC ATG GAC AGG AAT GGC CAG GAG AGC ATG CTC ACG
Ala Gln Asp Leu Phe Ser Val Tyr Met Asp Arg Asn Gly Gln Glu Ser Met Leu Thr
                                    200

                    640                          660                          680
                     |                            :                            :
CTG GGG GCC ATC GAC CCG TCC TAC TAC ACA GGG TCC CTG CAC TGG GTG CCC GTG ACA
Leu Gly Ala Ile Asp Pro Ser Tyr Tyr Thr Gly Ser Leu His Trp Val Pro Val Thr
210                                  220

                            700                          720                          740
                             |                            |                            |
GTG CAG CAG TAC TGG CAG TTC ACT GTG GAC AGT GTC ACC ATC AGC GGT GTG GTT GTG
Val Gln Gln Tyr Trp Gln Phe Thr Val Asp Ser Val Thr Ile Ser Gly Val Val Val
    230                                  240

                                760                          780
                                 |                            |
GCC TGT GAG GGT GGC TGT CAG GCC ATC CTG GAC ACG GGC ACC TCC AAG CTG GTC GGG
Ala Cys Glu Gly Gly Cys Gln Ala Ile Leu Asp Thr Gly Thr Ser Lys Leu Val Gly
        250                                  260

800                          820                          840
 |                            |                            |
CCC AGC AGC GAC ATC CTC AAC ATC CAG CAG GCC ATT GGA GCC ACA CAG AAC CAG TAC
Pro Ser Ser Asp Ile Leu Asn Ile Gln Gln Ala Ile Gly Ala Thr Gln Asn Gln Tyr
            270                                  280

            860                          880                          900
             |                            |                            |
GAT GAG TTT GAC ATC GAC TGC GAC AAC CTG AGC TAC ATG GCC ACT GTG GTC TTT GAG
Asp Glu Phe Asp Ile Asp Cys Asp Asn Leu Ser Tyr Met Pro Thr Val Val Phe Glu
                290                                  300

            920                          940                          960
             |                            |                            |
ATC AAT GGC AAA ATG TAC CCA CTG ACC CCC TCC GCC TAT ACC AGC CAA GAC CAG GGC
Ile Asn Gly Lys Met Tyr Pro Leu Thr Pro Ser Ala Tyr Thr Ser Gln Asp Gln Gly
                310                                  320

            980                          1000                         1020
             |                            |                            |
TTC TGT ACC AGT GGC TTC CAG AGT GAA AAT CAT TCC CAG AAA TGG ATC CTG GGG GAT
Phe Cys Thr Ser Gly Phe Gln Ser Glu Asn His Ser Gln Lys Trp Ile Leu Gly Asp
                330                                  340
```

```
                1040                          1060                          1080
                 |                             |                             |
GTT TTC ATC CGA GAG TAT TAC AGC GTC TTT GAC AGG GCC AAC AAC CTC GTG GGG CTG
Val Phe Ile Arg Glu Tyr Tyr Ser Val Phe Asp Arg Ala Asn Asn Leu Val Gly Leu
                             350                                          360

                1095
                 |
GCC AAA ACC ATC TGA
Ala Lys Thr Ile  *
                365
```

Linking of pTACR102B6 and pTACR103C2 can also be accomplished in the manner presented below. Namely, the linking employs plasmid pTACR5 as a joint portion which plasmid contains from the base site before *Kpn* I site (from No. 249 to 254) up to the site after *Bam* HI (from No. 1013 to 1018). pTACR5 was derivable from one of the ten prorennin cDNA containing clones obtained in process (4). DNA fragment (d) to be inserted was prepared by the treatment of pTACR 102B 6 with *Hind* III plus *Kpn* I. Fragment (e) containing 760 bases (plus chain side) was prepared by the treatment of pTACR 5 with *Kpn* I plus *Bam* HI through its partial digestion. Fragment (f) was prepared by the treatment of pTACR103C2 with *Bam* HI plus *Hind* III. The three fragments possess termini complementary to each other and these were annealed to form the linear DNA comprising (d)-(e)-(f). After annealing, the cyclic DNA pCR1001 was constructed by treatment with T4 DNA ligase.

The complete prorennin cDNA contained in plasmid pCR1001 was linked to the *lac* promoter region of *E. coli* lactose operon, with the frame fixed and thus pCR2001 was constructed. pCR2001 was inserted into *E. coli* C600 r⁻m⁻ by transformation. The plasmid-containing microorganism is designated as *E. coli* CR1 (r⁻m⁻, thr⁻, leu⁻, thi⁻; pCR2001) and has been deposited with the ATCC as ATCC No. 39170 (deposited on August 4, 1982).

The construction of pCR2001 is illustrated below in detail.

(1) Plasmid pBH20 [K. Itakura, T. Hirose, R. Crea, A. D. Riggs, H. L. Heyneker, F. Bolivar, H. W. Boyer; Science, *198*, 1056 (1977)] contains the *lac* promoter of *E. coli* lactose operon, the ribosome-binding site thereof and a part of N terminus of β-galactosides. Plasmid pBH20 was treated with *Eco* RI to form a linear DNA, which was then subjected to DNA polymerase I. The terminal single-stranded DNA portion thus becomes double-stranded. Decanucleotide containing *Bam* HI site was linked to the DNA at its both ends by the action of T4-DNA ligase and the DNA was further treated with *Bam* HI plus *Sal* I.

(2) Either pTACR 102 B6 or pCR1001 was treated with *Bam* HI plus *Kpn* I to produce a DNA fragment comprising from *Bam* HI site (No. 15) of the prorennin cDNA base sequence up to *Kpn* I site (No. 253).

(3) pCR 1001 was treated with *Kpn* I plus *Sal* I to produce a DNA fragment comprising from *Kpn* I site (no. 254) of the prorennin cDNA up to the terminal *Sal* I site.

Each DNA fragment obtained through steps (1), (2) and (3) was annealed together and, after annealing, the cyclic DNA pCR 2001 could be constructed by treatment with T4-DNA ligase.

(6) Production of Prorennin by *E. coli*

An aqueous medium (L broth) having the following composition was prepared:

| Ingredient | Grams/liter |
|---|---|
| Bacto-Tryptone (DIFCO) | 10 |
| Bacto Yeast extract (DIFCO) | 5 |
| Sodium chloride | 10 |
| pH 7.2 | |

The strain, *E. coli* CR1 was cultivated at 37°C under shaking in the neutrient medium containing 30 µg/ml of ampicillin, and 1 mM of IPTG (isopropylthio β-D-galactoside). Cells were harvested by centrifugation at 10,000 × g when propagation reached maximum. The Cells were then destroyed by *sonication* treatment and a cell-free extract was obtained. The prorennin protein in the extract was measured according to the radio-immunmoassay method using prorennin antibody labelled with ¹²⁵I. As a result of the measurement, it was found that about 1,000 moleculars of prorennin protein were produced per one cell of the host microorganism.

The extract was passed through a solid-state antibody column made of anti-prorennin Sepharose 4B, and the adsorbed protein was after elution tested on a SDS-polyacrylamide gel electrophenogram. The molecular weight of the product was thus confirmed to be similar to that of prorennin.

**Claims**

1. Microorganism E. coli ATCC 39170 containing plasmid pCR2001.
2. Plasmid pCR2001 as present in E. coli ATCC 39170.
3. A process for producing calf prorennin not containing the first 5 amino acids which comprises cultivating in a nutrient medium the genetically engineered microorganism E. coli ATCC 39170 as defined in claim 1.

**Patentansprüche**

1. Mikroorganismus E. coli ATCC 39170 mit dem Plasmid pCR2001.
2. Plasmid pCR2001 enthalten in E. coli ATCC 39170.
3. Verfahren zur Herstellung von Kalbprorennin ohne die ersten 5 Aminosäuren, gekennzeichnet durch die Züchtung des gentechnologisch hergestllten Mikroorganismus E coli ATCC 39170 gemäß Anspruch 1 in einem Nährmedium.

**Revendications**

1. Microorganisme E. coli ATCC 39170 contenant le plasmide pCR2001.
2. Plasmide pCR2001 tel que présent dans E. coli ATCC 39170.
3. Procédé de production de prochymosine de veau ne contenant pas les cinq premiers acides aminés, qui comprend la culture dans un milieu nutritif du microorganisme E. coli ATCC 39170 obtenu par génie génétique tel que défini dans la revendication 1.